(19) **Europäisches Patentamt**

**European Patent Office**

**Office européen des brevets**

(11) **EP 1 372 680 B1**

(12) **EUROPEAN PATENT SPECIFICATION**

(45) Date of publication and mention
of the grant of the patent:
**05.04.2006 Bulletin 2006/14**

(51) Int Cl.:
*A61K 36/00* (2006.01)   *A61P 21/02* (2006.01)

(21) Application number: **01929978.3**

(22) Date of filing: **29.03.2001**

(86) International application number:
**PCT/IN2001/000056**

(87) International publication number:
**WO 2002/078724 (10.10.2002 Gazette 2002/41)**

(54) **BIOLOGICALLY ACTIVE AQUEOUS FRACTION OF AN EXTRACT OBTAINED FROM A MANGROVE PLANT SALVADORA PERSICA L**

BIOLOGISCH WIRKSAM WÄSSRIGE FRAKTION EXTRAKT ERHALTEN AUS EINER MANGROVE PFLANZ SALVADORA PERSICA L

FRACTION AQUEUSE A ACTIVITE BIOLOGIQUE D'UN EXTRAIT VEGETAL DE MANGROVE SALVADORA PERSICA L.

(84) Designated Contracting States:
**DE FR GB**

(43) Date of publication of application:
**02.01.2004 Bulletin 2004/01**

(73) Proprietor: **COUNCIL OF SCIENTIFIC AND INDUSTRIAL RESEARCH**
**New Delhi 100 001 (IN)**

(72) Inventors:
• **GOSWAMI, Usha,**
**National Institute of Oceanography**
**Goa 403 004 (IN)**
• **FERNANDES, Nazarine,**
**Nat. Inst. of Oceanography**
**New Delhi 403 004 (IN)**

(74) Representative: **Portal, Gérard et al**
**Cabinet Beau de Loménie**
**158, rue de l'Université**
**75340 Paris Cédex 07 (FR)**

(56) References cited:
• **L. M. KAMIL ET AL.: "PHARMACOGNISTICAL AND PHYTOCHEMICAL STUDIES ON SALVADORA PERSICA L." JOURNAL OF PHARMACY AND PHARMACOLOGY, vol. 51, no. SUPPL., September 1999 (1999-09), page 227 XP001031401 LONDON, GB ISSN: 0022-3573**
• **S. T. EZMIRLY ET AL.: "SAUDI ARABIAN MEDICINAL PLANTS: SALVADORA PERSICA" PLANTA MEDICA, vol. 35, no. 2, 1978, pages 191-192, XP001031435 THIEME, STUTTGART, DE ISSN: 0032-0943**

## Description

### Technical Field

The invention relates to biologically active extracts obtained from the plant *Salvadora persica Linneaus* 1753. The invention also provides a process for obtaining the crude extract. Further the invention provides pharmaceutical compositions, exhibiting biological activity, especially tocolytic activity.

### Background Art

The associated mangrove plant, *Salvadora persica Linneaus,* 1753, belongs to the order Salvadoracea and are shrubs or small trees with white flowers frequent in degraded mangrove swamps and saline banks all over the west coast of India. Large numbers of marine plants have been examined for bioactive substances. Nazarine, F; Anita F; Rataboli, P.V.; Diniz D'Souza, R. S and Dhume; V.G., 1998 in Indian Journal of marine Sciences, 27: 499-501 have reported promising pharmacological activities in marine organisms from Indian waters.

There are several patents available from all over the world related to processes and compounds from nature for various purposes. Kwak; Wie-jong; Han; Chang-kyun; kum; Hwan-su; An; Jae-suk; Kum; Taek-soo, patented process of extracting and purifying biologically effective ingredients from combined medicinal plant and their extract composition (United States Patent No.5,910,307 published on June 8, 1999). D'Amelio; Frank S; Mirhorn; Youssef W. disclosed therapeutic composition and method for treating skin using extract from *Centipeda cunninghami* plant in US Patent No. 5,804,206 published on September 8, 1998. Zimmerman; Richard C.; Alber; Randall S.; Todd; James S.; Crews; Philip, isolated compound from the methanolic extract of the eelgrass *Zostrea marina* having significant antifouling aquatic properties (US Patent No.5,607,741 published on March 4, 1997).

Betulinic acid which is prepared from the compound Betulin has many pharmaceutical potentials. Pezzuto John M; Kim; Darrick S.H.L. disclosed methods of manufacturing betulinic acid from betulin (US Patent No.5,804,575 published on 8 September, 1998). The betulinic acid is intensively investigated as a potential therapeutic agent for a variety of diseases. Pezzuto; John, M; Das Gupta, Tapas K; Schmidt; Mary Lou; Kuzmanoff; Konrad Marc; Ling Indeck; Lydia and Kim; Darrick, S.H.L. (US Patent No.5,962,527 dated October 5, 1999). Pisha E, Chai H, Lee IS, Chagwedera TE, Framsworth NR, Cordell GA, Beecher CW, Fong H H, Kinghorn AD, Brown DM, in Nature medicine, 1 pages 1046-1051 (1995) discloses that betulinic acid has an unexpected selective anti-tumour activity against human melanoma e.g. MEL-1, MEL-2 and MEL-4. In addition Fujioka T, Kashiwada Y, Kilkuskie RE, Cosentino LM, Ballas LM, Jiang JB, Janzen WP, Chen IS, Lee KH. J.Nat.Prod., 57 (2) pages 243-247 (1994) discloses that betulinic acid has anti-HIV activity in H9 lymphocytic cells. These inventors mentioned that the researches directed to betulinic acid as therapeutic agent are hindered because the betulinic acid is available in very limited quantities and at a very high cost. Ramadoss, Sunder, Jaggi, Manu; Siddiqui; Mohammad Jamshed Ahmed in US Patent No.6,048,847 published on April 11, 2000 describes uses of betulinic acid and its derivatives for inhibiting cancer growth and a method of monitoring this. Kang; Raphael K.L.; Zyzak; Li Li; nakatsu; Tetsuo published flavoured product additives in US Patent No.5,948,460, dated 7 September, 1999 in which Ursolic acid was one of the compound amongst a group of three compounds which was added to a flavoured product to reduce aftertaste in the product and enhance its sweetness like in a diet drink. It was also used as a constituent in a preparation for inhibition of skin Tumorigenesis.

Herman, S (US Patent No.5,190,979 published on 2 March, 1993) disclosed lupeol also as a compound which can make pharmacologically active terpene ozonides which have medicinal value.

### Objects of the invention

The main object of the present invention is to provide a process for obtaining a crude extract from stem, leaves, and flowers of *Salvadora persica,* a commonly available shrub in mangrove swamps and screening to study its bioactivity.

Another object of the invention is to isolate naturally occurring compounds from the plant *Salvadora persica* and identify their molecular weights, molecular formulae, melting points and their structural formulae, which will be helpful in chemical synthesis of these compounds.

Yet another object of the invention is pharmacological screening of crude extract, its fractions and purified compounds to check that the activities shown by the crude extract and fractions is maintained throughout.

Another object of the invention is to provide pharmaceutical compositions containing extracts from the plant *Salvadora persica* and exhibiting biological activity, especially, tocolytic activity.

### Summary of the invention

The present invention seeks to overcome the drawbacks inherent in the prior art by providing the highly efficient and selective means for processing of active crude extract its fractionation, isolation and purification of the active compounds. Further the invention provides pharmaceutical compositions containing the extract obtained from the plant *Salvadora persica* and useful in relieving pains in the uterine muscles.

## Detailed description of the invention

The present invention discloses for the first time the methods of isolation, purification and pharmacological screening of all these above said commercially important compounds from a commonly available plant from mangrove swamps of the west coast of India. The said plant identified as *Salvadora persica,* is an associated mangrove plant. It is a shrub and the twigs with leaves of flowers can be hand picked. Even the crude extract and fractions exhibit therapeutic value. The important point is that the biological activity shown by the extract is maintained in the purified compounds such as Methyl palmitate and Betulin.

This disclosure points towards future potential clinical uses of the extract and fractions for treatment of diseases such as smooth muscle relaxant, bronchial asthma, renal colics and prevention of premature delivery. It further relates to the use of Methyl palmitate and Betulin in motion sickness, abdominal cramps.

The invention provides methods whereby biologically active crude extract of an associated mangrove plant identified as *Salvadora persica Linneaus* 1753 is prepared. *Salvadora persica Linneaus* (Salvadoraceae) are shrubs or small trees with white flowers frequent in degraded mangrove swamps and saline banks all over the west coast of India; west Asia. The process disclosed in the invention further relates to the extraction, fractionation and purification of active constituent metabolites of the said plant. The invention is also concerned with the spectral identification of the compounds such as β-amyrin (non-steroidalpolycyclic triterpene), Betulin, Ursolic acid (triterpenic acid), Methyl palmitate (Aliphatic Ester) and Lupeol (non-steroidalpolycylic triterpene). The invention also deals with molecular formulae, molecular weights, melting points and structural formulae of the said compounds. The invention provides a highly efficient and selective means for processing of active crude extract obtained from *Salvadora persica,* its fractionation, isolation and purification. As used herein, the terms fractionation means separating the crude extract. The term isolation and fractionation means separating the fraction into pure compounds.

The invention further relates to methods of screening pharmacological activities of the said compounds in mammalian tissues. The applicant has found that the crude extract obtained from the plant *Salvadora persica* can be separated into two fractions, i.e., chloroform and aqueous fractions. The aqueous fraction was found to exhibit tocolytic activity, which is described in detail in the present invention.

Accordingly, the invention provides a process of extracting and purifying biologically useful molecules from an associated mangrove plant which comprises the steps of:

i) collecting and processing the plant plants of *Salvadora persica,*
ii) preparing a crude extract from the plant parts of *Salvadora persica,*
iii) testing the crude extract using methods of pharmacology,
iv) fractionating the crude extract,
v) testing the fractions using methods of pharmacology,
vi) isolating the pure compounds by column chromatography,
vii) testing the pure compounds by using methods of pharmacology, and
viii) identifying the compounds by spectroscopy.

As said earlier, the aqueous fraction of the crude extract obtained from the mangrove plant *Salvadora persica* showed tocolytic activity, while the remaining 2 fractions (butanol and petroleum ether) were inactive.

The invention also provides the identification of the molecules from the spectral data. The molecular formulae of the five compounds is provided from the spectral data. The invention provides molecular weights of the molecules from EIMS. The structural formulae of the compounds is also provided from the spectral data.

Thus, a crude extract was obtained from the plant *Salvadora persica.* The crude extract was tested for its bioactivity and if found promising in terms of its pharmacological activity, it was fractionated using solvents with increasing polarity to obtain fractions such as petroleum ether, chloroform, butanol and aqueous. Each of these were also tested for their pharmacological activity.

The five compounds purified from the extract of the plant were β-amyrin (non - steroidalpolycyclic triterpene), Betulin, Ursolic acid (triterpenic acid), Methyl palmitate (Aliphatic Ester) and Lupeol (non-steroidalpolycyclic triterpene).

β-amyrin was found to be a non-steroidalpolycyclic triterpene with the following details:

$$\text{Molecular formula} \quad : C_{30} H_{50} O$$
$$\text{Molecular weight} \quad : 426$$

Table continued

| | |
|---|---|
| Melting point | : 160.degree C |

Another molecule found was betulin having:

| | |
|---|---|
| Molecular formula | : $C_{30} H_{50} O_2$ |
| Molecular weight | : 442 |
| Melting point | : 255 degree.C |

Ursolic acid (triterpenic acid) molecule was also found in the extract. It had:

| | |
|---|---|
| Molecular formula | : $C_{30} H_{48} O_3$ |
| Molecular weight | : 456 |
| Melting point | : 292 degree.C. |

Methyl palmitate (Aliphatic Ester) found in the extract had:

| | |
|---|---|
| Molecular formula | : $C_{16} H_{32} O_2$ |
| Molecular weight | : 256 |
| Melting point | : 30 degree.C |

Lupeol (non-steroidalpolycyclic triterpene) found in the extract had:

| | |
|---|---|
| Molecular formula | : $C_{30} H_{50} O$ |
| Molecular weight | : 426 |
| Melting point | : 215 degree. C |

One of the compounds Betulin can be used in manufacturing of betulinic acid (US Patent No.5.804.575 published on 8 September, 1998). Betulinic acid is intensively investigated as a potential therapeutic agent for a variety of diseases.

Ursolic acid is added to a flavoured product to reduce aftertaste in the product and enhance its sweetness for example in a diet drink. It was also used as a constituent in a preparation for inhibition of skin Tumorigenesis.

Extracts of some plants show vasoconstrictor and analgesic properties and also contain triterpenoid beta.-amyrin. These compositions for inhibiting the formation of unwanted skin pigmentation combine high tyrosinase blocking capabilities with stability in cosmetic preparations, absence of significant cytotoxic effects and synergy of action (US patent 5,773,014, 5,679,393). Bcta.-amyrin and lupeol are used as components for dimethylsterols in medical formulations (US Patent 4,808,574).

Methyl palmitate is a compound used in making alcohols as mentioned in US patent No. 6,049,013 published on April, 2000. Lupeol can be used as a component for several remedial medicines, insect repellants, distilleries anti tumour and chemical industries (US Patent No. 4,808,574; 5,962,527; 5,908,628).

Thus, the extract of *Salvadora persica* contained several compounds as listed above. The aqueous fraction of the extract of the said plant *Salvadora persica,* was then tested for its biological activity on guinea pigs. The Applicants, to their surprise found that the aqueous fraction exhibited excellent tocolytic activity.

Accordingly, the invention provides compositions containing the aqueous fraction of the extract obtained from *Salvadora persica,* optionally with conventional additives for relieving pains in the uterine muscles. The composition may contain about 10 μg of the extract. Thus, the aqueous fraction of the crude extract obtained from the plant *Salvadora persica* is a potential tocolytic agent. The compositions may be formulated in different physical forms, as may be required. The extract may be used as such or with conventional additives, physiologically acceptable carriers, preservatives, buffers, etc. as required.

Additionally, the invention provides the use, for the manufacture of a medicament for treating tocolytic conditions, of a therapeutically effective amount of extract obtained from *Salvadora persica.* The extract may be administered at a dosage level in the range of 50 μg/ml to 250μg/ml, in case of normal adults. The exact dosage will vary depending on the patient to be treated and will depend on factors such as requirements of the patient, severity of the condition being treated and the activity of the extract. The determination of optimum dosages for a particular patient is well-known to those skilled in the art.

**Detailed description of the accompanying drawings**

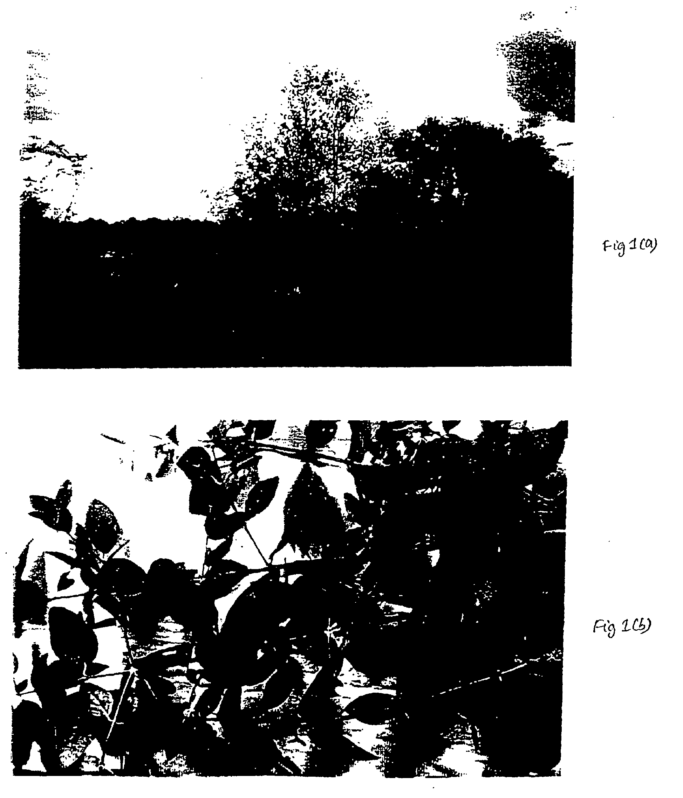

Fig 1(a)    shows the mangrove plant and
Fig 1(b)    shows the twig of the associated mangrove plant used.

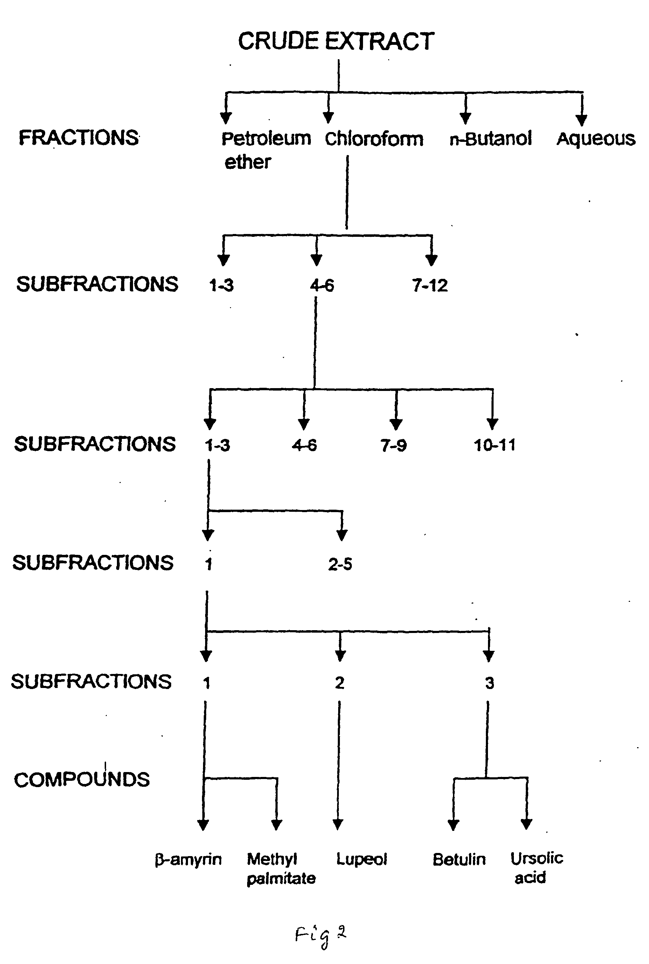

Fig 2:       shows the different fractions obtained from the crude extract of the plant *Salvadora persica.*

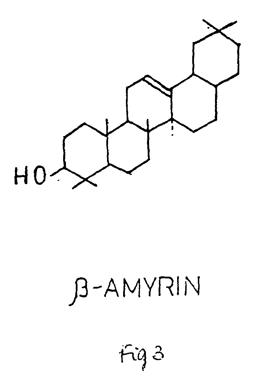

Fig.3:       shows the structural formula of β-amyrin (non - steroidalpolycyclic triterpene)

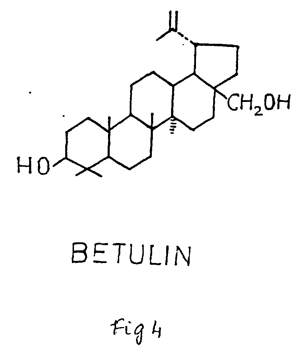

Fig.4:       Structural formula of Betulin,

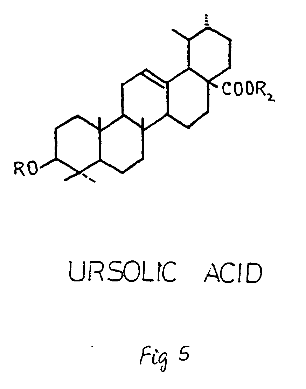

Fig 5:       Structural formula of Ursolic acid (triterpenic acid),

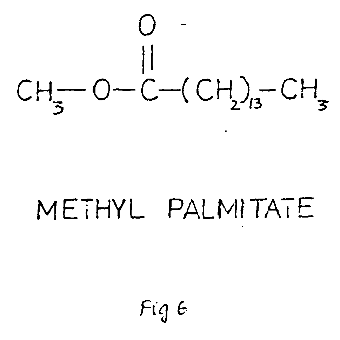

Fig.6:       Structural formula of Methyl palmitate (Aliphatic Ester)

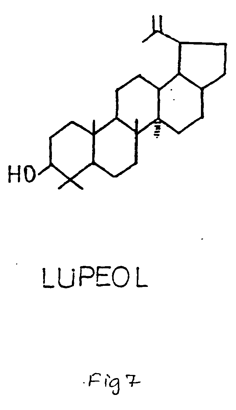

Fig.7:       Structural formula of Lupeol (non-steroidalpolycyclic triterpene).

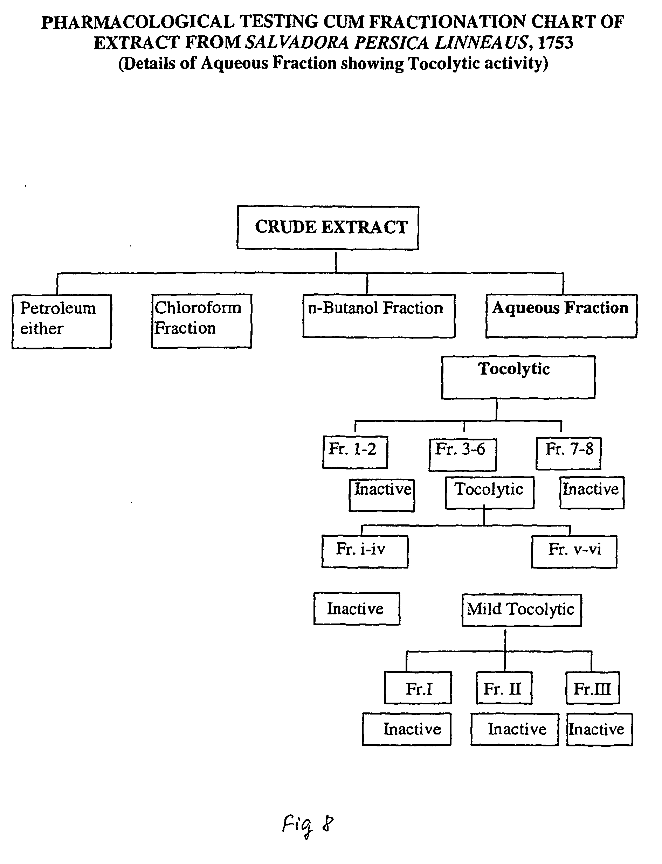

Fig.8        Fractionation chart of extract of *Salvadora persica*

The invention is described in detail and illustrated by the following examples which should not be construed as limitations on the inventive concept embodied herein.

**Example 1 : This example provides the Chemicals, Reagents, Apparatus used and their sources.**

| Name of reagent/chemicals | Company |
|---|---|
| Aqueous methanol | Sisco Research Laboratories Pvt Ltd. |
| Petroleum ether | Ranbaxy Fine Chemicals Ltd. |
| Chloroform | Sisco Research Laboratories Pvt Ltd. |
| Butanol | Sisco Research Laboratories Pvt Ltd. |
| Ethyl acetate | Sisco Research Laboratories Pvt Ltd. |
| Histamine acid phosphate | Blenkinsop & Co. Ltd |
| Acetylcholine chloride | Hopkin & Williams Ltd. |
| 5-Hydroxytryptamine creatine sulphate | Sigma Chemicals |
| Barium chloride | Apex Chemicals |
| Nicotine sulphate | BDH chemicals |
| Oxytocin | Parke Davis India Ltd. |
| Prostodin-PGF 2 alpha | Astra IDL Ltd. |

**APPARATUS:**

1.    Physiograph.
      Company: Biodevices,
      Ambala, India.
2.    Force Transducer
      Model No. T-305
      Co.: GRASS,USA
3.    Stimulator
      Model SS44
      Co. Biodevices, Ambala
4.    Polygraph
      Model 7
      Co. GRASS , USA.
5.    Force Transducer
      Model No. FT-03
      Co.: GRASS,USA
6.    Organ Bath
      Ambala,

India

**Example 2 :**

Collection of the mangrove plant *Salvadora persica* L from the coast of Goa, a state in India was along Ribandar, near the mouth of the Mandovi estuary, upstream (India). This species is ubiquitous to the coastal areas of Goa and was collected manually from the intertidal banks.

**Example 3:**

Processing of the collected mangroves were washed first with seawater followed by tap water. The undesired materials were sifted out while washing with tap water to get rid of the salts. The leaves, stems, and flowers of the associated mangrove plant were air dried. After drying, the plant material was cut into small pieces and immersed in the solvent 90% aqueous methanol for a week for extraction. Care was taken to ensure that these were properly soaked/ dipped in the solvent and checked for putrefaction.

**Example 4:**

Extraction and preparation of crude extract was carried out by cold percolation method at room temperature and by solvent evaporation at a water bath (temperature 50°C) under reduced pressure. This helps in protection of any heat labile metabolite present in it. Re-extraction was done twice until the extract was concentrated under vacuum to obtain the crude extract.

**Example 5**

**Fractionation of the crude extract:**

The crude extract was partitioned into petroleum ether, chloroform, n-butanol and aqueous fractions using a separating funnel. Petroleum ether was added to the extract in the separating funnel and separated out. Next, chloroform was added to the residue, mixed well and the lower layer separated. To the residue butanol was added and the top layer represented the butanol fraction and lower layer the aqueous fraction. Extraction of each fraction was done thrice and whenever there was emulsion sodium chloride was added for breaking the emulsion. Sodium sulphate was added to chloroform and butanol fractions to remove traces of water before concentration. All the fractions were concentrated in the same manner as the crude extract. These fractions were tested for the same pharmacological activity as the parent crude extract. Column chromatography for isolation of pure compound was done by repeated column chromatography and thin layer chromatography of the eluents. The TLC revealed compounds such as Beta-amyrin, betulin, ursolic acid and lupeol.

**Example 6**

To obtain the compounds Beta amyrin and betulin, separation by thin layer chromatography is carried out on 0.25mm thick silica gel plates (Qualigen). The eluent is an 90:10 (v/v) Petroleum ether/ethyl acetate mixture and the spots are developed by spraying with 5% $H_2SO_4$ solution and fixation by heating at 110.degree.C.

For compounds Ursolic acid and Methyl palmitate separation by thin layer chromatography were carried out on 0.25 mm thick silica gel plates (Qualigen). The eluent is an 85:15 (v/v) Petroleum ether/ethyl acetate mixture and the spots are developed by spraying with a 5% $H_2SO_4$ solution and fixation by heating at 110°C.

For the compound lupeol the separations by thin layer chromatography are carried out on 0.25mm thick silica gel plates (Qualigen). The eluent is an 75:25 (v/v) Petroleum ether/ethyl acetate mixture and the spots are developed by spraying with a 5% $H_2SO_4$ solution and fixation by heating at 110°C.

In the present invention, the active aqueous fraction of the mangrove plant S. persica , was column chromatographed over silica gel for the isolation of the active constituent. Elutes from the column with the same TLC profile were mixed and subjected to pharmacological testing. The active subfractions were further chromatographed till active pure compounds were obtained. Spots on thin layer chromatography (TLC) were visualised by using iodine vapours and spraying with methanolic sulphuric acid.

TLC was done on glass plates (20 X 20 cms) coated with a 0.25mm layer of TLC grade silica gel (Qualigens) activated at 110° C for 1 hour before use. The active aqueous fraction was passed through XAD-column and eluents were treated, as mentioned above, for the isolation and purification of active constituent metabolites.

The five compounds were identified on the basis of spectral data obtained at the Regional Sophisticated Instrumentation Centre (RSIC) by the following spectra :
$^1$HNMR for determining the proton environment of the molecule carried out on Bruker DPX-200 MHz.

Apparatus:    Bruker Spectrometer
              Model: DPX
              Co. Bruker

$^{13}$CNMR for carbon atoms Bruker DPV 300 MHz.
The compounds were identified from a comparison of their spectral data with those of similar compounds reported in literature.
Mass spectra (EIMS) electron impact Mass spectrometry for determining the molecular weights along with its fragmentation pattern were carried out on Mass spectrometer (E1/CIMS) Model D.300 JEOL,

Apparatus:    Mass Spectrometer (EIMS)
              Model: D-300
              Co. JEOL, Japan

## Example 6

Pharmacological testing of pure compounds
Standard drugs used were the following:

    Histamine acid phosphate (Blenkinsop & Co. Ltd.) on ileum.
    Acetylcholine chloride (Hopkin & Williams Ltd.) on ileum.
    5-Hydroxytryptamine creatinine sulphate (sigma Chemicals Co.) on gastrointestinal tract.
    Barium chloride (Apex Chemicals) on smooth muscle contraction.
    Nicotine sulphate (BDH Chemicals) on intestine as ganglion stimulant.
    Oxytocin (Parke Davis India Ltd) on uterus.
    Prostodin - $PGF_2$ (Astra IDL Ltd) on uterus.
    All other reagents used were of analytical grade.
    Tyrode was used on guinea-pig ileum and de Jalon's solution was used on guinea-pig uterus.
    Ringer-Locke physiological solution was used on guinea-pig atria table 3.
    All other reagents used were of analytical grade.

Physiological solutions used and various parameters:

    All physiological solutions were prepared fresh at the time of the experiment.
    pH: The pH of the various physiological salt solutions varied between 7.3 & 7.4. At lower pH the tonus of the preparation tends to decrease and is therefore liable to alter the effect of drugs.
    Temperature: In order to get consistent effects it was important to maintain the temperature of the bath solution at a specified level, because if the temperature is decreased below 37°C, the tonus of the intestine is increased, the contractions become smaller and the contraction and relaxation times increased.
    Air: Air or oxygen is needed for proper functioning of the tissues. Besides, providing oxygen to the tissues, the stream of gas bubbles also stirred the bath solution thereby facilitating diffusion of drugs added to the bath.
    The solution in the bath was changed frequently because prolonged aeration tends to alter the pH.

### *In vitro* experiments :

Female, virgin, guinea pigs weighing around 300 to 350 g, housed under uniform husbandry conditions (temperature 25 $\pm$ 1°C) were used. The animals were starved 24 hours prior to the experiment, only water was provided *ad libitum.*
The isolated guinea pig uterus was used to study the tocolytic activity.
For experiments upon isolated guinea pig, the bicornuate uterus was dissected out and freed of fat tissue. One horn was cut-off and kept in a shallow dish containing the physiological solution - de Jalon's fluid which was previously aerated with air. Air was preferred to oxygen as the tissue was thin and saturation was faster. Jalon's fluid comprises:

              Glucose              0.5 g

Table continued

| Sodium chloride | 9.0 g |
|---|---|
| Sodium bicarbonate | 0.5 g |
| Potassium chloride | 0.42 g |
| Calcium chloride | 0.06 g |

All were dissolved in 1000 ml water

The two ends were sutured. The lower end of the uterine strip was tied to a tissue holder and suspended in an organ bath of 10 ml capacity and the upper end being more sensitive, to the lever of the force transducer (FT 03) connected to a Grass Polygraph (Model 7). It was left to stabilize for 30 mins, renewing the physiological solution in the bath every 10 mins. The response of the uterus to different does of the extract (50 & 250 $\mu$g/ml) against standard uterine stimulants like oxytocin and PG $F_2 \alpha$ with a contact period of 60 seconds (was recorded on the polygraph). The tocolytic effect was evaluated by the following formula:

$$\% \text{ Inhibition} = \frac{X - Y}{X} \times 100$$

Wherein:

X = Height of standard contraction (mm)
Y = Height of standard contraction in presence of the extract (mm)

The relative in vitro tocolytic effect of *Salvadora persica* crude extract on guinea pig uterus is shown in the Table hereinbelow (mean of 5 readings):

| EXTRACT | TISSUE | SPASMOGENS | STANDARD INDUCED CONTRACTION (mm) 48 | DOSE 50mg /ml (mm) 25 | INHIBITION % DECREASE | DOSE 250 mg/ml 20 | INHIBITION % DECREASE |
|---|---|---|---|---|---|---|---|
| Crade Extract | Guines-pig Uterus | Oxy & PG | 48 | 25 | 48 | 20 | 58 |
| Chloroform | | Oxy & PG | 50 | 50 | 0 | 50 | 0 |
| BuOH | | Oxy & PG | 51 | 48 | 6 | 40 | 21 |
| Aqueous | | Oxy & PG | 48 | 35 | 27 | 25 | 48 |
| Fraction-1 | | Oxy & PG | 45 | 44 | 2 | 42 | 7 |
| Fraction-2 | | Oxy & PG | 48 | 48 | 0 | 45 | 6 |
| Fraction-3 | | Oxy & PG | 50 | 42 | 16 | 30 | 40 |
| Fraction-4 | | Oxy & PG | 50 | 44 | 12 | 28 | 44 |
| Fraction-5 | | Oxy & PG | 46 | 40 | 13 | 26 | 43 |
| Fraction-6 | | Oxy & PG | 48 | 40 | 17 | 24 | 50 |
| S fr I | | Oxy & PG | 49 | 52 | - | 52 | - |
| S fr II | | Oxy & PG | 50 | 50 | 0 | 52 | - |
| S fr III | | Oxy & PG | 48 | 40 | 20 | 32 | 40 |
| S fr IV | | Oxy & PG | 50 | 45 | 10 | 33 | 34 |
| S fr V | | Oxy & PG | 50 | 48 | 4 | 45 | 10 |
| S fr VI | | Oxy & PG | 52 | 50 | 4 | 50 | 4 |
| Potentiation<br>Abbreviations used:<br>OXT= Oxytocin:PG = PGR 2$\alpha$ | | | | | | | |

**Claims**

1. Use of a therapeutically effective amount of aqueous extract obtained from the plant *Salvadora persica,* optionally with conventional additives, for the manufacture of a medicament for relieving pains in the uterine muscles of a subject in need thereof.

2. A use as claimed in claim 1 wherein 3 to 10 μg/ml. amount of the extract is to be administered to the subject for a period of 10 minutes.

3. A use as claimed in claim 1 wherein the subject is a human or animal.

4. A process for the extraction and purification of biologically useful molecules from mangrove plant *Salvadora persica,* comprises the steps of:

   i) drying said plant;
   ii) cutting said dried plant into small pieces;
   iii) immersing said small plant pieces into about 95% aqueous methanol for about a week to obtain an extract;
   iv) evaporating said aqueous methanol from said obtained extract under reduced pressure at a temperature of about 55°C;
   v) cold-percolating said extract at room temperature;
   vi) repeating steps (iii)-(v) twice to obtain a concentrated crude extract;
   vii) fractionating said concentrated crude extract into a petroleum ether fraction, a chloroform fraction, an n-butanol fraction, and an aqueous fraction using separate funnels;
   viii) isolating said biologically useful molecules via chromatography; and
   ix) identifying said compounds via spectroscopy.

5. The method as claimed in claim 4, wherein said plant parts are selected from the group consisting of leaves, stems and flowers.

6. The method as claimed in claim 4, wherein said biologically useful molecules are further identified and purified by column chromatography and/or thin layer chromatography.

7. The method as claimed in claim 6, wherein silica gel plates are used in said thin layer chromatography.

8. The method as claimed in claim 4, wherein said biologically useful molecules are selected from the group consisting of β-amyrin, betulin, ursolic acid, methyl palmitate and lupeol.

**Patentansprüche**

1. Verwendung einer therapeutisch wirksamen Menge eines wässrigen Extrakts, das erhalten wird aus der Pflanze *Salvadora persica,* optional mit konventionellen Zusätzen, zur Herstellung eines Medikaments zum Lindern von Schmerzen in den Uterusmuskeln eines Subjekts, das dessen bedarf.

2. Verwendung nach Anspruch 1, worin 3 bis 10 μg/ml Menge des Extrakts an das Subjekt verabreicht werden muss für einen Zeitraum von 10 Minuten.

3. Verwendung nach Anspruch 1, worin das Subjekt ein Mensch oder Tier ist.

4. Verfahren zur Extraktion und Reinigung biologisch nützlicher Moleküle aus der Mangrovenpflanze *Salvadora persica,* welches die Schritte umfaßt von:

   i) Trocknen der Pflanze,
   ii) Schneiden der getrockneten Pflanze in kleine Stücke,
   iii) Eintauchen der kleinen Pflanzenstücke in etwa 95% wässriges Methanol für etwa eine Woche, um ein Extrakt zu erhalten,
   iv) Verdampfen des wässrigen Methanols aus dem erhaltenen Extrakt unter vermindertem Druck bei einer Temperatur von etwa 55°C,

v) Kaltfiltrieren des Extrakts bei Raumtemperatur,

vi) zweimal Wiederholen der Schritte iii) bis v), um ein konzentriertes Rohextrakt zu erhalten,

vii) Fraktionieren des konzentrierten Rohextrakts in eine Petroletherfraktion, eine Chloroformfraktion, eine n-Butanolfraktion und eine wässrige Fraktion unter Verwendung von Scheidetrichtern,

viii) Isolieren der biologisch nützlichen Moleküle über Chromatographie und

ix) Identifizieren der Verbindungen über Spektroskopie.

**5.** Verfahren nach Anspruch 4, worin die Pflanzenteile gewählt sind aus der Gruppe, die besteht aus Blättern, Stengeln und Blüten.

**6.** Verfahren nach Anspruch 4, worin die biologisch nützlichen Moleküle weiter identifiziert und gereinigt werden durch Säulenchromatographie und/oder Dünnschichtchromatographie.

**7.** Verfahren nach Anspruch 6, worin Silicagelplatten bei der Dünnschichtchromatographie verwendet werden.

**8.** Verfahren nach Anspruch 4, worin die biologisch nützlichen Moleküle gewählt sind aus der Gruppe, die besteht aus β-Amyrin, Betulin, Ursolsäure, Methylpalmitat und Lupeol.

## Revendications

**1.** Utilisation d'une quantité thérapeutiquement efficace d'un extrait aqueux obtenu à partir de la plante *Salvadora persica,* éventuellement avec des additifs classiques, pour la fabrication d'un médicament pour soulager les douleurs dans les muscles utérins d'un sujet en ayant besoin.

**2.** Utilisation telle que revendiquée dans la revendication 1, dans laquelle on doit administrer au sujet une quantité de 3 à 10 $\mu$g/ml de l'extrait sur une période de 10 minutes.

**3.** Utilisation telle que revendiquée dans la revendication 1, dans laquelle le sujet est un être humain ou un animal.

**4.** Procédé pour l'extraction et la purification de molécules biologiquement utiles à partir de la plante de mangrove *Salvadora persica* qui comprend les étapes consistant à :

(i) sécher ladite plante ;

(ii) couper ladite plante séchée en petits morceaux ;

(iii) immerger lesdits petits morceaux de plante dans du méthanol aqueux à environ 95% pendant environ une semaine afin d'obtenir un extrait ;

(iv) évaporer ledit méthanol aqueux à partir dudit extrait obtenu sous pression réduite à une température d'environ 55°C ;

(v) percoler à froid ledit extrait à température ambiante ;

(vi) répéter deux fois les étapes (iii) à (v) afin d'obtenir un extrait brut concentré ;

(vii) fractionner ledit extrait brut concentré en une fraction dans l'éther de pétrole, une fraction dans le chloroforme, une fraction dans le n-butanol et une fraction aqueuse en utilisant des ampoules à décanter ;

(viii) isoler lesdites molécules biologiquement utiles par chromatographie ; et

(ix) identifier lesdits composés par spectroscopie.

**5.** Procédé tel que revendiqué dans la revendication 4, dans lequel lesdites parties de plante sont choisies dans le groupe constitué par les feuilles, les tiges et les fleurs.

**6.** Procédé tel que revendiqué dans la revendication 4, dans lequel lesdites molécules biologiquement utiles sont en outre identifiées et purifiées par chromatographie sur colonne et/ou par chromatographie en couche mince.

**7.** Procédé tel que revendiqué dans la revendication 6, dans lequel on utilise des plaques de gel de silice dans ladite chromatographie en couche mince.

**8.** Procédé tel que revendiqué dans la revendication 4, dans lequel lesdites molécules biologiquement utiles sont choisies dans le groupe constitué par la β-amyrine, la bétuline, l'acide ursolique, le palmitate de méthyle et le lupéol.

Fig 1 (a)

Fig 1 (b)

# CRUDE EXTRACT

FRACTIONS    Petroleum  Chloroform  n-Butanol  Aqueous
ether

SUBFRACTIONS   1-3      4-6      7-12

SUBFRACTIONS   1-3    4-6    7-9    10-11

SUBFRACTIONS   1      2-5

SUBFRACTIONS   1      2      3

COMPOUNDS

β-amyrin  Methyl  Lupeol  Betulin  Ursolic
palmitate             acid

Fig 2

β-AMYRIN

Fig 3

14

BETULIN

Fig 4

URSOLIC ACID

Fig 5

$$CH_3-O-\overset{\overset{\textstyle O}{\textstyle ||}}{C}-(CH_2)_{13}-CH_3$$

METHYL PALMITATE

Fig 6

LUPEOL

Fig 7

**PHARMACOLOGICAL TESTING CUM FRACTIONATION CHART OF EXTRACT FROM *SALVADORA PERSICA LINNEAUS*, 1753
(Details of Aqueous Fraction showing Tocolytic activity)**

Fig 8